# EUROPEAN PATENT APPLICATION

(11) **EP 0 530 421 A1**
(43) Date of publication of application: **10.03.1993**
(21) Application number: 91402390.8
(22) Date of filing: 06.09.1991
(51) Int. Cl.: C12P 19/14

(54) **Method for the preparation of malto-oligosaccharide**

(71) Applicant: SHIN-ETSU CHEMICAL CO., LTD., Chiyoda-ku Tokyo (JP)
(72) Inventor: Kobayashi, Shiro, Sendai-shi, Miyagi-ken (JP); Shoda, Shin-ichiro, Sendai-shi, Miyagi-ken (JP); Kashiwa, Keita, Mitsubishi Dasei Onda-ryo, Yokohama-shi, Danagawa-ken (JP); Shimada, Junji, Sendai-shi, Miyagi-ken (JP)
(74) Representative: Armengaud Ainé, Alain

(57) **Abstract**

An efficient method for the preparation of malto-oligosaccharides, conventionally produced by the enzymatic hydrolysis of starch, from maltose is proposed in which fluorinated α-D-maltosyl, which can be obtained from maltose by a known procedure, is dissolved in a mixture of a water-soluble organic solvent, e.g., methyl alcohol, and a phosphate buffer solution and the solution is admixed with α-amylase so that an enzymatic reaction, which is inverse to the enzymatic hydrolysis of polysaccharides, takes place to form the oligosaccharides in a high yield.

## Description

The present invention relates to a method for the preparation of a malto-oligosaccharide or, more particularly, to a method for the preparation of a malto-oligosaccharide from maltose as the starting material.

As a definition, oligosaccharide is a saccharide compound of which the molecule is formed by a sequence of a small number of or, in particular, 2 to 10 monosaccharide units connected in series. Many of oligosaccharides have very unique properties not obtained in monosaccharides or polysaccharides consisting of a larger number of the monosaccharide units than in oligosaccharides so that oligosaccharides are expected to be a useful new material having applicability in a wide field. Unfortunately, preparation of an oligosaccharide is usually not a simple matter and no efficient method generally applicable to the preparation thereof is known. Accordingly, it is eagerly desired to establish a novel and efficient method for the preparation of oligosaccharides in general.

Malto-oligosaccharide is one of such oligosaccharides and widely used as an additive in foods, medicinal material, substrate material for various clinical enzymatic tests and the like. The conventional method for the preparation of malto-oligosaccharides is to partially hydrolyze starch as a starting material by using amylase. This method of enzymatic hydrolysis has several problems and disadvantages. For example, malto-oligosaccharides having a desired number of the monosaccharide units can be obtained only by very exactly controlling the reaction conditions. Further, the amylase used for the enzymatic hydrolysis of starch should be a special variant of the enzyme in order that the hydrolysis can be partial as desired. Moreover, heptamer or higher malto-oligosaccharides cannot be obtained by this method of enzymatic hydrolysis of starch. Accordingly, it is eagerly desired to develop a method for the preparation of malto-oligosaccharides free from these problems.

The inventors have conducted extensive investigations to solve the above described problems arriving at a discovery that malto-oligosaccharides can be prepared very efficiently by the enzymatic reaction of fluorinated α-D-maltosyl, which can be derived from maltose, with α-amylase. While α-amylase is known to act as an enzyme for the hydrolysis of starch, the reaction here taking place is an inverse reaction of hydrolysis to increase the number of the monosaccharide units.

Thus, the method of the present invention for the preparation of a malto-oligosaccharide, which is expressed by the structural formula 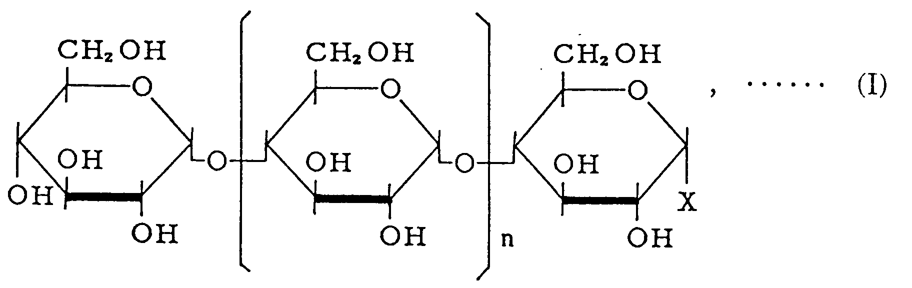
in which n is a positive integer of 2 or larger and X is a hydroxyl group or a fluorine atom, comprises: dissolving fluorinated ∝₋D₋ maltosyl expressed by the structural formula 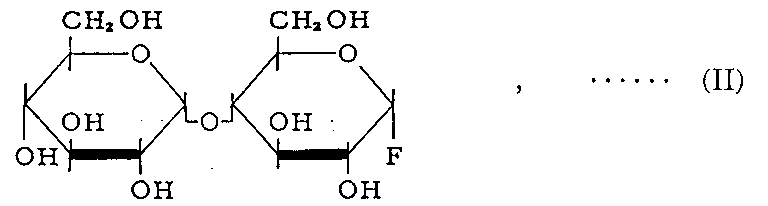
in a mixture of a water-soluble organic solvent and a buffer solution to effect an enzymatic reaction in the presence of α-amylase.

The starting material in the above mentioned enzymatic reaction is fluorinated α-D-maltosyl which can be prepared by the synthetic route described below consisting of the steps (i) to (vi), of which the steps for the preparation of the intermediate (iv) are based on the teaching given by E.J. Hehre, et al. in Journal of Biological Chemistry, volume 254, No. 13, page 5942 (1979) and the steps leading to the fluorinated α-D-maltosyl from this intermediate (iv) are based on the teaching given by Y.V. Veznig, et al. in Carbohydrates Research, volume 132, page 339 (1983). Needless to say, the fluorinated α-D-maltosyl used in the inventive method as the starting material is never limited to the product of this synthetic route but can be any one obtained by other methods. 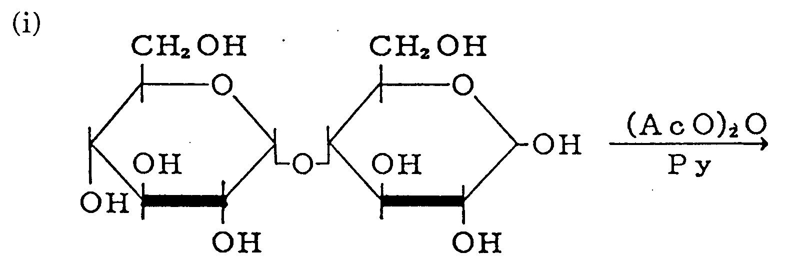 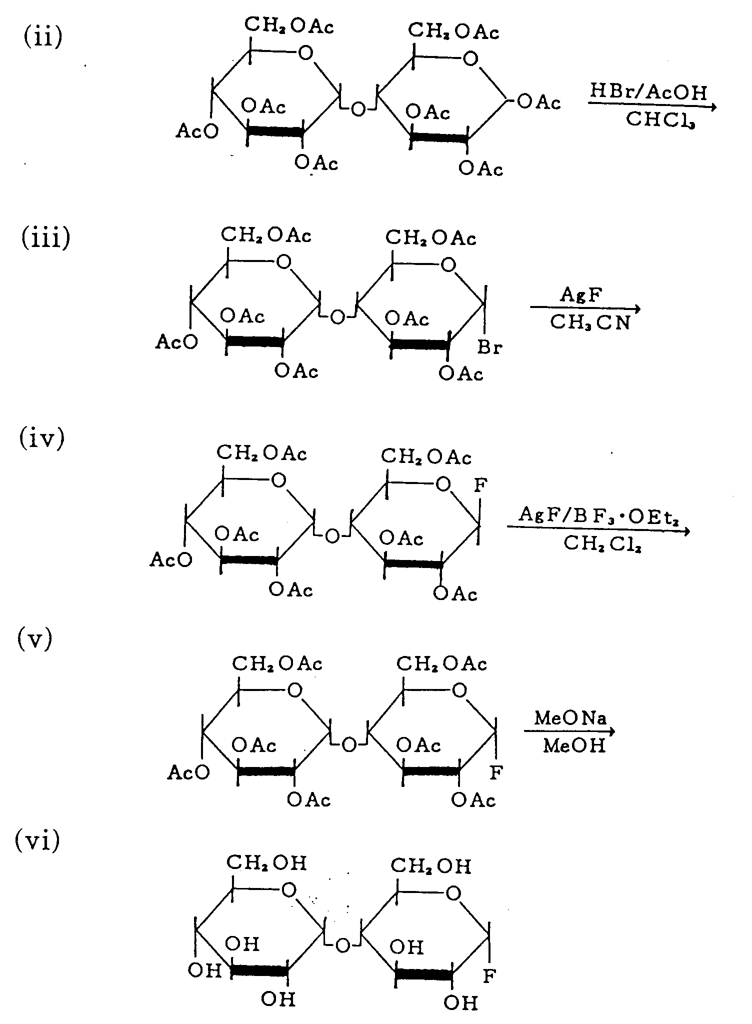
One of the characteristic features of the inventive method is in the use of a mixture of a water-soluble organic solvent and a buffer solution. Examples of suitable water-soluble organic solvents include methyl alcohol, ethyl alcohol, acetonitrile, acetone and the like, of which methyl alcohol is preferred in respect of the high efficiency for the conversion of the starting material into the product. The buffer solution is preferably a phosphate buffer solution. The mixing ratio of the organic solvent and the buffer solution has some influences on the efficiency of conversion of the starting material to the product and the molecular weight ditribution of the resulting malto-oligosaccharides but it is usually in the range from 1:1 to 2:1 by volume. The fluorinated α-D-maltosyl is dissolved in this mixed solvent in a concentration of, usually, from 0.001 to 0.1 g/ml or, preferably, around 0.01 g/ml.

The reaction temperature is not particularly limitative provided that the α-amylase can exhibit the enzymatic activity but it is usual to conduct the reaction at a temperature in the range from 20 to 50 °C. The reaction time should be selected depending on the desired molecular weight distribution in the product of the malto-oligosaccharides.

Following is a description of a typical procedure for practicing the inventive method. For example, the fluorinated α-D-maltosyl is dissolved in a mixture of a water-soluble organic solvent and a buffer solution in the above mentioned concentration to form a substrate solution to which a solution of α-amylase in the same buffer solution is added. The reaction mixture thus formed is agitated at a specified temperature for a specified length of time followed by heating of the mixture to terminate the reaction by deactivating the enzyme. The reaction mixture is then filtrated to remove precipitates of the deactivated enzyme and the filtrate is concentrated by evaporating the solvent to give the product in a crude form.

In the following, the inentive method is described in more detail by way of examples which, however, should never be construed to limit the scope of the invention in any way.

### Example 1.

A 100 mg portion of fluorinated α-D-maltosyl was taken in a test tube to which 2.6 ml of a 0.05 M phosphate buffer solution having a pH of 7.0 and 6 ml of methyl alcohol were added and then a solution of 5 mg of α-amylase obtained from *Aspergillus oryzae* (Type X-A, a product by Sigma Co.) in 0.4 ml of the same buffer solution as above was added thereto to start the enzymatic reaction which was continued under agitation for 1 hour at room temperature. The volume ratio of methyl alcohol to the buffer solution was 2:1.

After the end of the above mentioned reaction time, the reaction mixture in the test tube was heated for 10 minutes under reflux to deactivate the enzyme followed by filtration to remove the precipitates of the deactivated enzyme and concentration of the filtrate by evaporating the solvent to give a reaction product.

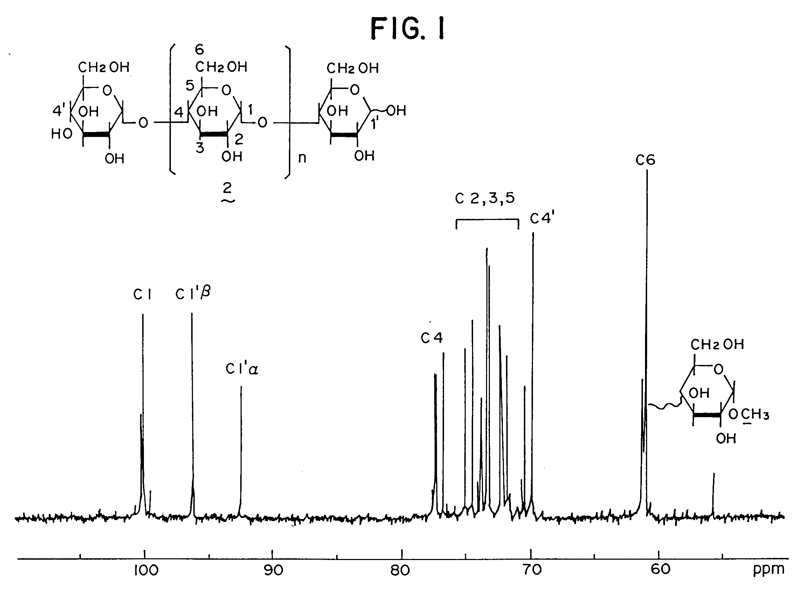

The reaction product was dissolved in heavy water and subjected to the measurement of the ¹³C-NMR spectrum. As is clear from the NMR spectrum obtained by this measurement as is shown in Figure 1, each of the peaks could be assigned to the carbon atoms forming the pyranose ring of the oligosaccharides and the carbon atoms in the glucose unit at the reduced or non-reduced terminal (C₁'β, C₁'α, C₄'). This result supports formation of malto-oligosaccharides. The peak at 55.7 ppm is assignable to the methyl group of the methoxide bonded to the 1-position of the reduced terminal presumably due to partial transfer of the glucosyl groups at the reduced terminals to the methyl alcohol as the solvent.

The reaction product was then subjected to high-performance liquid chromatography to examine the molecular weight distribution. The results are shown in Table 1 below. The conversion of the starting fluorinated α-D-maltosyl into the malto-oligosaccharides was 100%. The conditions of the chromatographic analysis were as follows.
Column: Hibar Lichrosorb RP-18 (manufactured by Merck Co.)
Column size: 4 mm diameter x 250 mm length
Loading: a solution of 1.7 mg product in 1 ml water introduced
Eluant: water
Elution velocity: 1 ml/minute
Detection: RI
Identification: by comparison with commercial products of standard samples

### Example 2.

A 100 mg portion of fluorinated α-D-maltosyl was taken in a test tube to which 4.1 ml of a 0.05 M phosphate buffer solution having a pH of 7.0 and 4.5 ml of methyl alcohol were added and then a solution of 5 mg of α-amylase in 0.4 ml of the same buffer solution as above was added thereto to start the enzymatic reaction. The volume ratio of methyl alcohol to the buffer solution was 1:1. The reaction procedure was substantially the same as in Example 1. Conversion of the starting fluorinated α-D-maltosyl to the malto-oligosaccharides was 100%. The molecular weight distribution in the product determined by the high-performance liquid chromatography was as shown in Table 1.

### Example 3.

A 100 mg portion of fluorinated α-D-maltosyl was taken in a test tube to which 1.1 ml of a 0.05 M phosphate buffer solution having a pH of 7.0 and 7.5 ml of methyl alcohol were added and then a solution of 5 mg of α-amylase in 0.4 ml of the same buffer solution as above was added thereto to start the enzymatic reaction. The volume ratio of methyl alcohol to the buffer solution was 5:1. The reaction procedure was substantially the same as in Example 1. Conversion of the starting fluorinated α-D-maltosyl to the malto-oligosaccharides was 27%. The molecular weight distribution in the product determined by the high-performance liquid chromatography was as shown in Table 1.

### Example 4.

A 100 mg portion of fluorinated α-D-maltosyl was taken in a test tube to which 2.6 ml of a 0.05 M phosphate buffer solution having a pH of 7.0 and 6 ml of acetone were added and then a solution of 5 mg of α-amylase in 0.4 ml of the same buffer solution as above was added thereto to start the enzymatic reaction. The volume ratio of acetone to the buffer solution was 2:1. The reaction procedure was substantially the same as in Example 1. Conversion of the starting fluorinated α-D-maltosyl to the malto-oligosaccharides was 73%. The molecular weight distribution in the product determined by the high-performance liquid chromatography was as shown in Table 1.

**Table 1**

| Example No. | % Frsaction of oligosaccharides | | | | | | |
|---|---|---|---|---|---|---|---|
| | G₂ | G₃ | G₄ | G₅ | G₆ | G₇ | G₄F |
| 1 | 12 | 25 | 28 | 13 | 14 | 5 | 4 |
| 2 | 21 | 33 | 22 | 11 | 5 | 0 | 7 |
| 3 | 43 | 40 | 11 | 0 | 0 | 0 | 5 |
| 4 | 37 | 24 | 26 | 7 | 2 | 0 | 3 |
| (Note) G₂: maltose; G₃: maltotriose; G₄: tetramer; G₅: pentamer; G₆: hexamer; G₇: heptamer; G₄F: α-D-maltotetraosyl | | | | | | | |

## Claims

1. A method for the preparation of a malto-oligosaccharide which comprises: dissolving fluorinated α-D-maltosyl in a mixture of a water-soluble organic solvent and a buffer solution to effect an enzymatic reaction in the presence of α-amylase.

2. The method for the preparation of a malto-oligosaccharide as claimed in claim 1 in which the water-soluble organic solvent is selected from the group consisting of methyl alcohol, ethyl alcohol, acetonitrile and acetone.

3. The method for the preparation of a malto-oligosaccharide as claimed in claim 2 in which the water-soluble organic solvent is methyl alcohol.

4. The method for the preparation of a malto-oligosaccharide as claimed in claim 1 in which the buffer solution is a phosphate buffer solution.

5. The method for the preparation of a malto-oligosaccharide as claimed in claim 1 in which the mixing ratio of the water-soluble organic solvent and the buffer solution is in the range from 1:1 to 2:1 by volume.

6. The method for the preparation of a malto-oligosaccharide as claimed in claim 1 in which the concentration of the fluorinated α-D-maltosyl in the mixture of the water-soluble organic solvent and the buffer solution is in the range from 0.001 to 0.1 g/ml.

7. The method for the preparation of a malto-oligosaccharide as claimed in claim 1 in which the reaction temperature is in the range from 20 to 50 °C.
